# EUROPÄISCHE PATENTANMELDUNG

(11) **EP 2 065 059 A1**
(43) Veröffentlichungstag der Anmeldung: **03.06.2009**
(21) Anmeldenummer: 07023147.7
(22) Anmeldetag: 29.11.2007
(51) Int. Cl.: A61L 2/18, A61B 19/00

(54) **Verfahren und Vorrichtung zur Reinigung von medizinischen Geräten, insbesondere von Endoskopen**

(71) Anmelder: HAMMANN Wasser Kommunal Ingenieurgesellschaft für kommunale Dienstleistungen mbH, 76855 Annweiler am Trifels (DE); Hytecon GmbH, 32052 Herford (DE)
(72) Erfinder: Hammann, Hans-Gerd, 76855 Annweiler am Trifels (DE); Kolch, Andreas, Dr., 32052 Herford (DE)
(74) Vertreter: Lenzing, Andreas

(57) **Zusammenfassung**

Die Erfindung betrifft ein Verfahren zur innenseitigen Reinigung von wenigstens ein Lumen aufweisenden medizinischen Geräten, mit folgenden Schritten:
a) Anschließen des Geräts an eine Reinigungsvorrichtung, wobei ein druckseitiges Ende über eine Druckleitung mit einer Reinigungsflüssigkeitsquelle und ein niederdruckseitiges Ende mit einem Ablauf verbunden wird,
b) Durchleiten der Reinigungsflüssigkeit durch das Lumen, wobei
c) in die Druckleitung mehrere Volumina eines Druckgases eingespeist werden, die die Reinigungsflüssigkeit beschleunigt durch das Lumen befördern.

## Beschreibung

Die vorliegende Erfindung betrifft ein Verfahren und eine Vorrichtung mit den Merkmalen des Oberbegriffs des Anspruchs 1 bzw. des Anspruchs 10.

Bei medizinischen Geräten mit Hohlräumen, die bei der Benutzung mit Körperflüssigkeiten in Berührung kommen können, ist die Reinigung und Sterilisation ein Wesentlicher, vor der Benutzung erforderlicher Vorgang. Endoskope in flexibler und starrer Bauform weisen ein inneres Lumen auf, durch das Lichtquellen, Werkzeuge und dergleichen eingeführt werden können. Dieses Lumen kommt bei der Benutzung beispielsweise mit Blut des Patienten in Kontakt. Wenn dieses Blut koaguliert oder verkrustet, bildet sich ein Belag im Innern des Endoskops, dessen Entfernung mit den herkömmlichen Reinigungsmaschinen mit erheblichem Aufwand verbunden ist. Die betroffenen Endoskope müssen gegebenenfalls manuell mit Bürsten oder dergleichen vorgereinigt werden, bevor sie in eine handelsübliche Reinigungsmaschine zur Aufbereitung eingesetzt werden.

Nur mit Flüssigkeiten arbeitende Reinigungsvorrichtungen werden von verschiedenen Herstellern angeboten. Eine derartige Reinigungsmaschine spült, reinigt und sterilisiert das Endoskop. Dieser Prozess muss hinsichtlich der erzielten Sterilisation zuverlässig und sicher ablaufen. Es ist aber auch gewünscht, diesen Prozess möglichst kurz zu gestalten, damit die Verfügbarkeit der Endoskope möglichst hoch ist.

Aus der WO2004/112975A1 ist ein Verfahren bekannt, bei dem einem Druckluftstrom Flüssigkeit in Form von kleinen Tröpfchen zugefügt wird. Diese Tröpfchen sollen Ablagerungen im Innern des Endoskops mechanisch lösen oder abtragen. Problematisch könnte dabei sein, dass die Tröpfchen nach Kontakt mit der Wandung des Lumens nicht wieder in den Luftstrom eintreten, sondern dort verbleiben und weiter stromabwärts liegende Verkrustungen nicht erreichen.

Es ist deshalb Aufgabe der vorliegenden Erfindung, ein Reinigungsverfahren und eine Reinigungsvorrichtung dahingehend zu verbessern, dass auf die manuelle Vorreinigung in vielen Fällen oder ganz verzichtet werden kann und dennoch der Reinigungsprozess innerhalb der gleichen Zeit abläuft oder sogar verkürzt wird.

Diese Aufgabe wird von einem Verfahren mit den Merkmalen des Anspruchs 1 und einer Vorrichtung mit den Merkmalen des Anspruchs 10 gelöst.

Weil das Verfahren zur innenseitigen Reinigung von wenigstens ein Lumen aufweisenden medizinischen Geräten folgende Schritte aufweist:
a) Anschließen des Geräts an eine Reinigungsvorrichtung, wobei ein druckseitiges Ende über eine Druckleitung mit einer Reinigungsflüssigkeitsquelle und ein niederdruckseitiges Ende mit einem Ablauf verbunden wird,
b) Durchleiten der Reinigungsflüssigkeit durch das Lumen,und
c) Einspeisen mehrerer Volumina eines Druckgases in die Druckleitung, die die Reinigungsflüssigkeit beschleunigt durch das Lumen befördern, wird ein besonders gutes Reinigungsergebnis bei Anhaftungen und Biofilmen in dem zu bearbeitenden Gerät erzielt, so dass in nahezu allen Fällen eine mechanische Vorreinigung entfallen kann.

Wenn die Reinigungsflüssigkeit mit einem ersten Druck in das Lumen eingespeist wird und dass das Druckgas mit einem zweiten Druck eingespeist wird, der höher ist als der erste Druck, kann die Einspeisung ohne eine aktive Unterbrechung der Flüssigkeitszufuhr erfolgen, was einen Steuerungseingriff einspart.

Vorteilhaft insbesondere für flexible Endoskope ist es, wenn der zweite Druck 100 mbar bis 1000 mbar über dem ersten Druck liegt. Das Druckgas kann gefilterte und gereinigte Luft sein.

Eine Ausbildung von Gasblasen in dem Lumen, die zu einer exponentiellen Expansion und zu einer entsprechenden Beschleuinigung der Reinigungsflüssigkeit für, ergibt sich, wenn während der Einspeisung der Volumina der Zustrom der Reinigungsflüssigkeit aktiv oder durch passive Mittel wie Rückschlagventile gestoppt ist.

Vorzugsweise werden die Volumina im Schritt c) jeweils getaktet für eine Zeit zwischen 100 ms und 1000 ms eingespeist.Dabei können die Volumina im Schritt c) mit einer Frequenz von 0,5/s bis 2/s eingespeist werden, so dass sich eine schnelle Abfolge von Flüssigkeit und Gas im Lumen ergibt, aber die Flüssigkeitspakete kompakt bleiben und nicht zu klein werden.

Ein gutes Reinigungsergebnis stellt sich ein, wenn die Schritte b) und c) für einen Zeitraum von 5 min bis 10 min durchgeführt werden.

Nach dem intermittierenden Spülen mit Flüssigkeits- und Gasvolumina kann ein konventionelles Spülen des Lumens mit Reinigungsflüssigkeit und Desinfektionslösung ohne Einspeisung von Gas durchgeführt werden, wie es aus dem Stand der Technik bekannt ist.

Weil eine Vorrichtung zur innenseitigen Reinigung von wenigstens ein Lumen aufweisenden medizinischen Geräten, mit
- wenigstens einem druckseitigen Anschluss für das Lumen des zu reinigenden Geräts, welcher mit einer Quelle von mit Druck beaufschlagter Reinigungsflüssigkeit verbindbar ist,
- wenigstens einem niederdruckseitigen Anschluss, der mit einem Auslauf in Verbindung steht,
- einer Steuerung, die für eine Einspeisung der Reinigungsflüssigkeit in das Lumen eingerichtet ist, und Mittel zum gesteuerten Einspeisen eines Druckgases in die Druckseite vorgesehen sind, kann damit das erfindungsgemäße Verfahren materialschonend und effektiv durchgeführt werden.

Wenn die Mittel wenigstens ein Steuerventil und zwei Rückschlagventile umfassen, wobei ein Rückschlagventil zwischen und der Quelle der Reinigungsflüssigkeit und dem druckseitigen Anschluss und das zweite Rückschlagventil der Druckgasquelle und dem druckseitigen Anschluss derart eingeschaltet sind, dass kein Reinigungsfluid von dem Anschluss in Richtung auf die Quellen strömen kann, kann die Vorrichtung als Modul in eine am Markt verfügbare Reinigungsmaschine integriert werden.

Nachfolgend wird ein Ausführungsbeispiel der vorliegenden Erfindung anhand der Zeichnung beschrieben. Es zeigt:

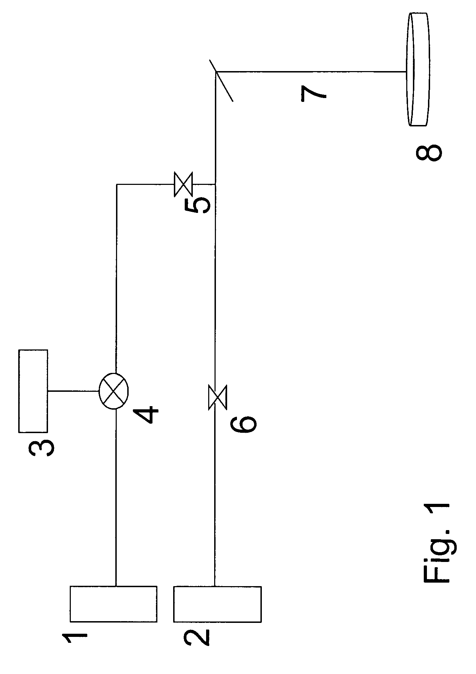

- Fig.1:: ein Schaltschema für eine erfindungsgemäße Vorrich- tung zur Reinigung von Endoskopen.

In der Fig. 1 ist nach Art eines Schaltbilds schematisch der Aufbau einer Vorrichtung zur Durchführung des erfindungsgemäßen Verfahrens dargestellt. Die Vorrichtung umfasst eine Luftversorgung 1 und eine Wasserversorgung 2. Ein Impulsformer 3 ist elektrisch mit einem Magnetventil 4 verbunden, um Luft aus der Luftversorgung 1 in Richtung auf eine erste Rückschlagklappe 5 zu leiten. Das Wasser, gegebenenfalls angereichert mit Reinigungsmitteln und Desinfektionsmitteln, wird durch eine zweite Rückschlagklappe 6 geleitet. Der Ausgang der beiden Rückschlagklappen 5 und 6 ist mit der Spülstrecke 7 verbunden, in die ein Endoskop eingesetzt ist. Das freie Ende der Spülstrecke 7 mündet in eine Vorrichtung 8 zum Druckabbau, beispielsweise in einen Zyklonabscheider.

Die Luftversorgung 1 umfasst weiter einen Regler für den zur Verfügung gestellten Luftdruck. Entsprechend weist auch die Wasserversorgung 2 einen Regler für den Wasserdruck auf. Die Luftversorgung 1 kann entweder eine vorhandene Druckluftversorgung sein, oder durch einen zusätzlichen Kompressor zur Verfügung gestellt werden. Die Druckluft sollte für den Einsatz im medizinischen Bereich entsprechend aufbereitet sein, z. B. durch einen Sterilfilter.

Für den Einsatz der genannten Komponenten im Bereich der Endoskopieaufbereitung werden diese so gewählt, dass sie problemlos in bestehende Vorrichtungen für die Aufbereitung von Endoskopen integriert werden können. Vorzugsweise wird eine Reinigungsvorrichtung nach dem Stand der Technik durch Einbau der erforderlichen Komponenten erfindungsgemäß umgerüstet. Dabei ist es an sich ausreichend, wenn die Komponenten 1, 3, 4 und 5 sowie gegebenenfalls die Rückschlagklappe 6 in ein bestehendes System eingebaut werden, und zwar wie in der Fig. 1 dargestellt zwischen der Wasserversorgung und der Spülstrecke.

Das erfindungsgemäße Verfahren läuft folgendermaßen ab:

Zunächst wird das zu reinigende Endoskop in die Reinigungsvorrichtung eingesetzt, indem es einerseits an einen Adapter auf der Druckseite der Spülstrecke 7 angeschlossen wird und andererseits mit einem ähnlichen Adapter an den Eingang des Zyklonabscheiders. Sodann wird zunächst Wasser aus der Wasserversorgung durch die Rückschlagklappe 6 in das Endoskop 7 eingespeist. Die Einspeisung erfolgt dabei mit einem Druck und einer Flussrate, die aus dem Stand der Technik bekannt sind, so dass das Endoskop nicht beschädigt wird. Die Luftversorgung 1 stellt gefilterte, gereinigte und somit sterile Druckluft mit einem Druck von mehreren 100 mbar bereit. Der Impulsformer 3 hält zunächst das Magnetventil 4 geschlossen. Der aus der Wasserversorgung 2 durch die Rückschlagklappe 6 strömende Wasserstrom steht gegen die Rückschlagklappe 5 und hält diese geschlossen, so dass kein Spülwasser in die Strecke zwischen dem Magnetventil 4 und der Rückschlagklappe 5 eindringen kann. Je nach Anwendung steuert nun der Impulsformer 3 das Magnetventil 4 an, so dass Luft mit einem höheren Druck als das an der Rückschlagklappe 5 anstehende Wasser durch das Magnetventil 4 strömen kann. Die Rückschlagklappe 5 öffnet auf Grund des höheren Luftdrucks, so dass Luft in die Leitung zwischen der Rückschlagklappe 6 und der Spülstrecke 7 eintreten kann. Die Rückschlagklappe 6 bewirkt, dass das Wasser nicht durch den Luftdruck rückwärts in die Wasserversorgung 2 gedrängt wird. Die Luft tritt dann in die Spülstrecke 7 ein und treibt das darin befindliche Wasser vor sich her. Der Druckabfall zum drucklosen Ende im Zyklonabscheider 8 bewirkt, dass sich die Luft zunehmend ausdehnt, was zu einer Beschleunigung des in der Spülstrecke 7 befindlichen Wassers führt. Der Spülvorgang wird dadurch zu einem hochturbulenten Prozess.

Um eine regelmäßige Wiederholung dieses Vorgangs zu gewährleisten und um auch regelmäßig Spülwasser in die Spülstrecke 7 gelangen zu lassen, steuert der Impulsformer 3 das Magnetventil 4 getaktet an. Die Impulse, während derer das Magnetventil 4 geöffnet ist, haben vorzugsweise eine Impulsdauer von 100 ms bis 1 s. Die bevorzugte Wiederholfrequenz liegt zwischen 500 ms und 2s.

Bei einem derartigen Betrieb stellt sich in der Spülstrecke 7 eine Abfolge von Luftvolumina und Wasserpaketen ein, wobei die Wasserpakete zum drucklosen Ende des Endoskops hin stark beschleunigt werden. Die induzierten Turbulenzen und auch die rasche Abfolge der Phasenübergänge trocken/nass bewirken dabei eine zuverlässige mechanische Reinigung der inneren Oberfläche sowohl von Verkrustungen und Koagulationen als auch von Biofilmen, die sich im Laufe der Zeit in Endoskopen bilden könnten.

Die Dauer dieses Prozesses wird in der Praxis nicht mehr als 5 - 10 Minuten beanspruchen. Der Prozess läuft auch bei relativ geringen Temperaturen von bis zu 35°C zuverlässig ab. Dabei wird ein befriedigendes Ergebnis bereits mit reinem Wasser erreicht. Geeignete Reinigungsmittel und Desinfektionsmittel sind so auszuwählen, dass kein übermäßiges Schäumen entsteht. Dies ist bei den üblichen Reinigungsmitteln unproblematisch.

Das insoweit beschriebene Verfahren zur Reinigung mit Luftimpulsen, die in die Spülstrecke eingespeist werden, kann die manuelle, mechanische Vorreinigung ersetzen. Hierdurch wird sowohl die gesamte Bearbeitungszeit für die Reinigung des Endoskops verringert, wodurch die Verfügbarkeit der Endoskope steigt, als auch die Gefahr von mechanischen Beschädigungen im Zuge der Reinigung verringert.

Das erfindungsgemäße ist als Vorreinigungsstufe ausgebildet. Nach dieser Vorreinigung erfolgt eine weitere Reinigung und Sterilisation in an sich bekannter Weise. Dabei kann auf Grund der gründlichen Vorreinigung der nachfolgende Reinigungsprozess gegenüber einer Kombination aus manueller Reinigung und maschinellem Reinigungsprozess verkürzt werden, denn die Entfernung von Verkrustungen und Biofilmen ist bei dem erfindungsgemäßen Verfahren genauso zuverlässig wie bei manueller Reinigung. Es ist zu erwarten, dass mit dem erfindungsgemäßen Prozess der Zeitaufwand für den gesamten Prozess (Vorreinigung mit Luftimpulsen, konventionelle Hauptreinigung und konventionelle Sterilisation) bei gleichem Ergebnis kürzer sein kann als der bisherige Prozess.

Die erfindungsgemäße Vorrichtung kann in einer an sich konventionellen Reinigungsvorrichtung für die Endoskopieaufbereitung bestehen, die durch Einschleifen der oben genannten Komponenten zur Einspeisung von Luftimpulsen in die Spülleitung ergänzt wird. Gegebenenfalls ist außer den Ventilen und den Rückschlagklappen eine eigene Druckluftversorgung erforderlich, wenn diese im Gerät oder in der Umgebung nicht verfügbar ist. Der Impulsformer 3 kann bei geeigneter Programmierung in der Steuerung der Reinigungsvorrichtung selbst realisiert werden.

Vorzugweise wird das erfindungsgemäße Verfahren angewendet in Maschinen zur Aufbereitung von flexiblen und starren Endoskopen, in Dialysegeräten und in zahnärztlichen Einheiten.

## Patentansprüche

1. Verfahren zur innenseitigen Reinigung von wenigstens ein Lumen aufweisenden medizinischen Geräten, mit folgenden Schritten:
a) Anschließen des Geräts an eine Reinigungsvorrichtung, wobei ein druckseitiges Ende über eine Druckleitung mit einer Reinigungsflüssigkeitsquelle und ein niederdruckseitiges Ende mit einem Ablauf verbunden wird,
b) Durchleiten der Reinigungsflüssigkeit durch das Lumen, **dadurch gekennzeichnet, dass**
c) in die Druckleitung mehrere Volumina eines Druckgases eingespeist werden, die die Reinigungsflüssigkeit beschleunigt durch das Lumen befördern.

2. Verfahren nach Anspruch 1, **dadurch gekennzeichnet, dass** die Reinigungsflüssigkeit mit einem ersten Druck in das Lumen eingespeist wird und dass das Druckgas mit einem zweiten Druck eingespeist wird, der höher ist als der erste Druck.

3. Verfahren nach Anspruch 2, **dadurch gekennzeichnet, dass** der zweite Druck 100 mbar bis 1000 mbar über dem ersten Druck liegt.

4. Verfahren nach einem der vorhergehenden Ansprüche, **dadurch gekennzeichnet, dass** das Druckgas gefilterte und gereinigte Luft ist.

5. Verfahren nach einem der vorhergehenden Ansprüche, **dadurch gekennzeichnet, dass** während der Einspeisung der Volumina der Zustrom der Reinigungsflüssigkeit gestoppt ist.

6. Verfahren nach einem der vorhergehenden Ansprüche, **dadurch gekennzeichnet, dass** die Volumina im Schritt c) jeweils für eine Zeit zwischen 100 ms und 1000 ms eingespeist werden.

7. Verfahren nach einem der vorhergehenden Ansprüche, **dadurch gekennzeichnet, dass** die Volumina im Schritt c) mit einer Frequenz von 0,5 /s bis 2 /s eingespeist werden.

8. Verfahren nach einem der vorhergehenden Ansprüche, **dadurch gekennzeichnet, dass** die Schritte b) und c) für einen Zeitraum von 5 min bis 10 min durchgeführt werden.

9. Verfahren nach einem der vorhergehenden Ansprüche, **dadurch gekennzeichnet, dass** nach dem Schritt c) ein Spülen des Lumens mit Reinigungsflüssigkeit ohne Einspeisung von Gas durchgeführt wird.

10. Vorrichtung zur innenseitigen Reinigung von wenigstens ein Lumen aufweisenden medizinischen Geräten, mit
- wenigstens einem druckseitigen Anschluss für das Lumen des zu reinigenden Geräts, welcher mit einer Quelle von mit Druck beaufschlagter Reinigungsflüssigkeit verbindbar ist,
- wenigstens einem niederdruckseitigen Anschluss, der mit einem Auslauf in Verbindung steht,
- einer Steuerung, die für eine Einspeisung der Reinigungsflüssigkeit in das Lumen eingerichtet ist,
**dadurch gekennzeichnet, dass** Mittel zum gesteuerten Einspeisen eines Druckgases in die Druckseite vorgesehen sind.

11. Vorrichtung nach Anspruch 10, **dadurch gekennzeichnet, dass** die Mittel wenigstens ein Steuerventil und zwei Rückschlagventile umfassen, wobei ein Rückschlagventil zwischen und der Quelle der Reinigungsflüssigkeit und dem druckseitigen Anschluss und das zweite Rückschlagventil der Druckgasquelle und dem druckseitigen Anschluss derart eingeschaltet sind, dass kein Reinigungsfluid von dem Anschluss in Richtung auf die Quellen strömen kann.
